# EUROPEAN PATENT APPLICATION

(11) **EP 4 530 260 A1**
(43) Date of publication of application: **02.04.2025**
(21) Application number: 24202551.8
(22) Date of filing: 25.09.2024
(51) Int. Cl.: C02F 1/78, A61L 2/18, A61L 2/20, B01F 23/23, B01F 25/00, B60S 3/04

(54) **WASHING SYSTEM**

(30) Priority: 29.09.2023 ES 202330817
(71) Applicant: Istobal, S.A., 46250 La Alcudia (Valencia) (ES)
(72) Inventor: TOMÁS ALFARO, Rafael, 46250 LA ALCUDIA (Valencia) (ES)
(74) Representative: Ungria López, Javier

(57) **Abstract**

The present invention relates to an ozonation system configured to ozonise a fluid and a washing system, preferably for vehicles, which is configured to mix said ozonated fluid obtained by the ozonation system according to the present invention using a chemical product to obtain a washing fluid for washing said vehicle.

In addition, the present invention also relates to a method of washing a vehicle by means of a washing system according to the present invention.

## Description

### OBJECT OF THE INVENTION

The present invention relates to an ozonation system configured to ozonise a fluid and a washing system, preferably for vehicles, which is configured to mix said ozonised fluid obtained by the ozonation system according to the present invention using a chemical product to obtain a washing fluid for washing said vehicle.

In addition, the present invention also relates to a method of washing a vehicle by means of a washing system according to the present invention.

### BACKGROUND OF THE INVENTION

Professional washing equipment is well known in the current state of the art, particularly washing equipment for vehicles that operates based on the Sinner Circle principle.

This principle is related to obtaining an efficient washing procedure for a vehicle based on the parameters of washing time, the chemical product used in combination with water, the mechanical energy required during the washing procedure, for example, using brushes or washing fluid applied at high pressure on the vehicle being washed, both of which form a washing fluid, and the temperature of said washing fluid.

This circle is in constant equilibrium in an efficient washing procedure so that increasing one of the parameters mentioned above automatically readjusts the other parameters to maintain this equilibrium.

In particular, various chemicals known in the state of the art that are used for cleaning cars have evolved into biodegradable, non-toxic chemicals with a low impact on the environment, which can degrade naturally and thus reduce their negative environmental impact.

However, despite the progress made, high-efficiency cleaning procedures still require the widespread use of chemicals in large quantities. This means that high-efficiency cleaning procedures require a constant supply of chemicals in order to function properly, which has the disadvantages of high costs and environmental impact, and the logistical difficulties involved in providing a constant supply of these chemicals to the required locations.

Additionally, transporting these chemicals carries the risk of spills or leaks, which in turn cause environmental damage, damage to facilities, as well as health and safety risks for the workers involved.

Moreover, the relationship between the temperature of the washing fluid and an optimal washing procedure requires the washing fluid to be at a high temperature, which increases the effectiveness of the cleaning procedure and the removal of contaminants adhered to the vehicle surface, such as grease, oil or insect residues.

There are known specialised systems in the current state of the art that generate and apply hot water in procedures for washing vehicles, which incorporate heaters to increase the temperature of the washing fluid. However, this type of system requires bigger facilities as well as more power to operate the system, which increases the costs of the washing procedure.

In addition, applying a high-temperature washing fluid may damage the surface of the vehicle since the high-temperature washing fluid may overheat and potentially damage paint, plastics and other sensitive materials.

Additionally, using such a washing fluid at high temperatures poses a risk to the safety of workers and users involved in the washing procedure.

Moreover, a washing fluid at high temperatures also aids the proliferation of bacteria, increasing the risk of contagious diseases for the users participating in the washing procedure.

As an additional solution to the known state of the art, ozone can be used in cleaning and disinfection procedures to eliminate odours and/or disinfect surfaces in contact with users, particularly in vehicle interiors.

Documents WO2023063488A1 and WO2022106740A1 describe the use of ozone in car cleaning, while CN1736887A relates to the treatment of waste water from car washing machines.

However, the disclosures in these documents show that the use of ozone in high concentrations has the drawback of potentially forming bubbles in the washing system, which has a negative impact on the high-pressure pumps used in the washing processes due to the cavitation produced, which drastically reduces the lifetime of these pumps and thus of the washing system.

In addition, a high concentration of ozone that is poorly dissolved in water can generate oxidising environments that can deteriorate the facilities, as well as pose a risk to the health of the workers involved in the procedure due to the presence of said ozone in the air.

The present invention makes it possible to solve the aforementioned problems, which are currently unsolved in the state of the art, by means of a high efficiency fluid ozonation system, as well as a high efficiency vehicle washing system and washing method, which are described below.

### DISCLOSURE OF THE INVENTION

In order to obtain a highly efficient system and procedure for washing a vehicle, which in turn makes it possible to avoid the disadvantages mentioned above, the present invention relates to an ozonation system configured to ozonise a fluid and a washing system, preferably for vehicles, which is configured to mix said ozonated fluid obtained by the ozonation system according to the present invention, using a chemical product to obtain a washing fluid for washing said vehicle.

Finally, the present invention also relates to a method of washing a vehicle using a washing system according to the present invention.

Thus, the first inventive aspect is related to an *ozonation system configured to ozonate a fluid, characterised in that the ozonation system comprises:*
- *storage means configured to store fluids, which comprise:*
   - *a first inlet configured to allow a first incoming fluid to enter the storage means,*
   - *a second inlet configured to allow a second incoming fluid to enter the storage means,*
   - *a first outlet configured to allow an outgoing fluid to exit the storage means; and*
   - *control means, which in turn comprise a processor, a memory and measuring means configured to measure ozone concentration (CO3), wherein the control means are configured to control the ozone concentration (CO3) of the outgoing fluid,*
- *circulating means, which are in fluidic communication with the storage means and configured to draw an outgoing fluid stream through the first outlet of the storage means,*
- *mixing means, which comprise:*
   - *a first inlet port, which is in fluidic communication with the circulating means and configured to introduce an outgoing fluid stream into said mixing means,*
   - *a second inlet port configured to introduce an ozone stream, and*
   - *a first outlet port configured to allow an ozonated fluid stream to exit the port,*
      *wherein the mixing means are configured to introduce an ozone stream into the outgoing fluid stream, obtaining an ozonated fluid, and*
      *wherein the first outlet port of the mixing means is in fluidic communication with the second inlet port of the storage means,*
   *wherein the control means are adapted to compare the ozone concentration (CO3) of the outgoing fluid exiting the storage means with a predetermined ozone concentration value (VCO3), and perform the following operations:*
- *if CO3 < VCO3, the control means;*
   - *direct an outgoing fluid stream through the circulating means to the mixing means, obtaining an ozonated fluid with a higher ozone concentration, and*
   - *introduce the ozonated fluid stream with higher ozone concentration (CO3C) into the storage means through the second inlet of said storage means,*
- *if CO3 = VCO3, the outgoing fluid is a final ozonated fluid and the control means prevent said final ozonated fluid from exiting the storage means by blocking the first output of the storage means.*

The present system has the advantage that it makes it possible to obtain an oxidising fluid, the outgoing fluid, based on enriching the second incoming fluid with ozone.

In a particular embodiment, the second incoming fluid is the ozonated fluid.

In particular, the present ozonation system makes it possible to start from the mixture of a first incoming fluid and a second incoming fluid with an ozone concentration and, through the control means, particularly through the measuring means in said control means, to measure the concentration of ozone dissolved in the fluid in question, and thus to control the ozone concentration of said mixture, enriching it in successive steps carried out by the elements of said ozonation system.

Thus, the second incoming fluid entering the system has a higher ozone concentration than the starting fluid, i.e., the initial mixture of the first incoming fluid and the second incoming fluid.

In a particular embodiment, the measuring means are configured to perform a direct measurement of the concentration of ozone diluted in a fluid. Such measuring means are, for example, a probe, preferably a probe configured to measure the oxidation reduction (redox) potential of the fluid, or an ORP probe.

In a particular embodiment, the measuring means are located on the storage means, being in contact with the fluid contained in the storage means.

In order to obtain higher concentrations of ozone, the outgoing fluid is taken as a starting point and recirculated through the present ozonation system in order to enrich it with more ozone.

This produces an outgoing fluid with an optimal ozone concentration value, i.e., a final ozonated fluid. This optimum ozone concentration value is predetermined based on the intended application of the volume of final ozonated fluid.

Therefore, when the above condition is met, where the ozone concentration of the outgoing fluid and the predetermined ozone concentration value of the outgoing fluid are equal, no outgoing fluid exits the storage means.

In a particular embodiment, the predetermined ozone concentration value of the outgoing fluid is preferably between 0.5 - 4 ppm of ozone, more preferably between 0.5 - 3 ppm, and most preferably between 1 - 2 ppm.

The ozonation system comprises storage means configured to house a total volume of fluid. This total volume is the sum of the first incoming fluid and the second incoming fluid, forming the mixture of the outgoing fluid totalling a volume that is the sum of said fluids.

In a particular embodiment, the storage means also comprise diffusion means configured to obtain a homogeneous outgoing fluid by mixing the first incoming fluid and the second incoming fluid. These diffusion means have the advantage that they make it possible to better dissolve the second incoming fluid with the first incoming fluid, allowing the present ozonation system to produce highly efficient ozonation.

In a particular embodiment, both the first inlet and the second inlet of the storage means are placed in such a way as to promote the mixture of the first and second incoming fluids.

In a particular embodiment, the first inlet is located on the upper part of the storage means.

In a particular embodiment, the second inlet is located on the lower part of the storage means.

A particular embodiment has the advantage that optimal mixing is obtained inside the storage means by combining the position of the first inlet on the upper part of the storage means and the second inlet on the lower part of the storage means.

In a particular embodiment, the diffusion means also comprise additional elements located on the first inlet and/or the second inlet of the storage means, which make it possible to mix more efficiently the contents of the storage means. In a particular embodiment, these additional elements are a diffuser block, for example.

In a particular embodiment, the first incoming fluid of the storage means is water, which has the advantage of dissolving ozone better.

In a particular embodiment, at least one of the first inlet, second inlet and first outlet of the storage means comprises an adjustable opening valve, preferably a self-regulating opening valve, configured to connect to the processor of the control means of the storage means.

This has the advantage of making it possible to control the inflow and/or outflow volumes of the various fluids to and from the storage means by regulating the cross-section allowing fluid to pass. This opening is regulated on the basis of the amount of fluid contained in the storage means.

In a particular embodiment, the storage means is a container or tank.

In a particular embodiment, the storage means also comprise a second outlet configured to allow an outgoing fluid to exit said storage means. In a particular embodiment, the outgoing fluid stream extracted through the second outlet of the storage means forms the incoming fluid of an external system.

The ozonation system comprises circulating means, which allow the outgoing fluid contained in the storage means to circulate through the rest of the circuit of the present ozonation system via the first outlet of said storage means.

In this way, the circulating means allow an outgoing fluid stream to be transferred from inside the storage means through the first outlet of the storage means to the mixing means.

In a particular embodiment, the circulating means are a pump, preferably of the centrifugal type.

The ozonation system comprises mixing means, which make it possible to mix the outgoing fluid exiting the storage means with a volume of ozone, obtaining a fluid with a higher content of ozone than the starting outgoing fluid before it is introduced into the mixing means.

This has the advantage of making it possible to constantly and completely dissolve the ozone gas in the fluid.

In a particular embodiment, the mixing means are a static mixer. In a particular embodiment, the mixing means also comprise a first injector located on the first inlet port and configured to inject the outgoing fluid stream into the mixing means through said first inlet port.

In a particular embodiment, the mixing means also comprise a second injector configured to inject the ozonated fluid stream into the mixing means through said first inlet port.

Injecting the ozone fluid has the advantage of making it possible to mix it more efficiently with the outgoing fluid. Furthermore, injecting said outgoing fluid into the injection means also makes it possible to mix more efficiently the fluids introduced into the mixing means.

In a particular embodiment, the mixing means comprise a Venturi system, which in turn comprises a first injector and a second injector that have the advantage of making it possible to draw the desired flow rate and pressure in a controlled manner.

In a particular embodiment, the mixing means also comprise a recirculating system configured to recirculate at least a portion of the ozonated fluid stream from the first outlet port to the first inlet port of said mixing means.

This has the advantage of making it possible to enrich the outgoing fluid with higher ozone concentrations, as said concentration increases from the recirculating or bypass system.

In a particular embodiment, the ozone stream introduced through the second inlet port of the mixing means is ozone in its gaseous state.

In a particular embodiment, the ozonation system also comprises ozone generating means in fluidic communication with the second inlet port of the mixing means and configured to generate the ozone stream to be introduced into said mixing means.

In addition, the ozone generating means are in communication with the control means in the storage means, so that the control means make it possible to deactivate the ozone generating means in the event the set condition CO3 = VCO3 is met. In this way, when the above condition is met, where the ozone concentration of the outgoing fluid and the predetermined value of ozone concentration in the outgoing fluid are equal, the control means prevent the outgoing fluid from exiting the storage means while avoiding the generation of additional volumes of ozone, which is unnecessary as the desired concentration has already been reached.

This makes it possible to generate a high purity ozone stream, preferably from 98% oxygen.

In a particular embodiment, the ozone generating means comprise a reactor, which in turn comprises a first access and a second access,
wherein:
- the first access is configured to allow the entry of a pure oxygen stream,
- the second access is configured to allow the exit of an ozone stream, and
- the reactor is configured to obtain an ozone stream from the pure oxygen stream, which is configured to be introduced into the mixing means.

The reactor has the advantage that is makes it possible to obtain a highly concentrated ozone stream, which preferably has between 92 - 98% purity.

In a particular embodiment, the ozone generating means also comprise a concentrator, which in turn comprises a concentrator inlet and a concentrator outlet, wherein:
- the concentrator inlet is configured to allow an air stream at ambient conditions to enter,
- the concentrator outlet is in fluidic communication with the second reactor access and configured to allow a stream, preferably a pure oxygen stream, to exit,
wherein the concentrator is configured to obtain a pure oxygen stream from the air stream at ambient conditions, which is configured to be introduced into the reactor of the ozone generating means.

In a particular embodiment, the ozonation system also comprises a first fluid flow means located between the circulating means and the mixing means, wherein said first fluid flow means are configured to ensure the first incoming fluid and the second incoming fluid are continuously fed into the storage means.

Additionally, the first fluid flow means are also configured to prevent the ozone stream generated by the ozone generating means from increasing above the volume of ozone required for operating the ozonation system and thus to obtain the predetermined target value of ozone concentration.

This has the advantage of preventing the circulating means from operating under a vacuum, which in turn prevents the volume of ozone generated by the ozone generating means from increasing in the ozonation system, accumulating in the second inlet port of the mixing means and reaching ozone concentration values in the ozonated fluid that are harmful and unsafe.

In a particular embodiment, the ozonation system also comprises a second fluid flow means located on the second inlet port of the mixing means and configured to regulate access of the ozone stream through the second inlet port of the mixing means.

In a particular embodiment, the second fluid flow means are a solenoid valve, preferably a three-way valve and/or a non-return valve.

This has the advantage of preventing the fluid channelled through the circulating means from reaching the ozone generating means so said fluid follows the correct path through the circuit described without being diverted along secondary paths, such as the path followed by the ozone gas stream generated in the ozone generating means.

A second inventive aspect is related to a *washing system configured to wash a vehicle, characterised in that it comprises:*
- *washing means,*
- *a first washing inlet,*
- *a second washing inlet,*
- *a washing outlet comprising a nozzle,*
- *an ozonation system according to the first inventive aspect where the storage means comprise a second outlet, and*
- *feeds located between the second outlet of the storage means and the first washing inlet, which are in fluidic communication with both,*
*wherein:*
- *the feeds are configured to draw a final ozonated fluid stream from the second outlet of the storage means of the ozonation system and introduce it into the washing means through the first washing inlet,*
- *the second washing inlet is configured to allow the entry of a chemical product,*
- *the washing means are configured to mix the final ozonated fluid stream and the chemical product to obtain a washing fluid; and*
- *the washing outlet nozzle is configured to allow the exit of a washing fluid stream.*

In this way, the feeds make it possible to transfer a final ozonated fluid stream from inside the storage means through the second outlet of the storage means to the washing means.

In a particular embodiment, the feed is a pump, preferably of the centrifugal type.

The present washing system has the advantage that it makes it possible to obtain an oxidising washing fluid with a higher washing capacity.

In a particular embodiment, the washing system also comprises pressurising means configured to increase the pressure at the outlet of the washing fluid stream.

This has the advantage that it makes it possible to apply the washing fluid mechanically to the vehicle, which improves the performance of said washing fluid.

A third inventive aspect is related to a *method of washing a vehicle using a washing system according to the second inventive aspect, characterised in that it comprises the following steps:*
*a) providing the washing means with a final ozonated fluid stream through the first washing inlet,*
*b) providing the washing means with a chemical product stream,*
*c) mixing the final ozonated fluid stream and the chemical product stream using the washing means,*
*d) obtaining a washing fluid stream, and*
*e) washing the vehicle with the washing fluid stream extracted through the washing outlet nozzle.*

The present method has the advantage that it makes it possible to wash a vehicle more efficiently based on using a more effective washing fluid and an optimised method of applying said washing fluid.

In a particular embodiment, the chemical product is soap, preferably liquid soap, which allows for a more effective washing procedure.

In a particular embodiment, the final ozonated fluid of step a) is obtained by an ozonation system according to the first inventive aspect where the storage means comprise a second outlet, which is configured to operate continuously until the outgoing fluid meets the condition CO3 = VCO3.

This has the advantage of obtaining an optimal washing fluid, preferably for washing a vehicle.

### BRIEF DESCRIPTION OF THE FIGURES

To complete the description of the invention and in order to help understand the characteristics thereof, according to preferred embodiments thereof, it is accompanied by a set of drawings in which the figures described below have been represented, for purely illustrative purposes and should not be construed as limiting:
Figure 1 shows a schematic view of an ozonation system according to a first embodiment of the invention.
Figure 2 shows a schematic view of a vehicle washing system according to a first embodiment of the invention.

### DESCRIPTION OF A PREFERRED EMBODIMENT OF THE INVENTION

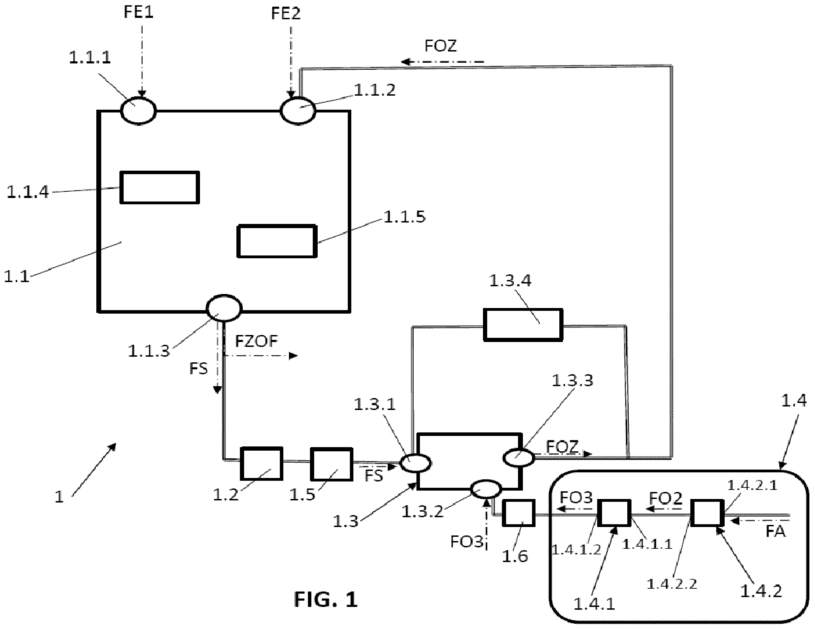

Figure 1 shows a schematic view of an ozonation system (1) according to a first embodiment of the invention, wherein said ozonation system (1) comprises storage means (1.1), in particular a tank (1.1).

Said storage means (1.1) comprise a first inlet (1.1.1) allowing the entry of a first incoming fluid (FE1), in this case water, and a second inlet (1.1.2) allowing the entry of a second incoming fluid (FE2), or ozonated fluid (FOZ), in this case ozonated water.

Figure 1 does not show the preferred locations of the first inlet (1.1.1) and the second inlet (1.1.2) of the storage means (1.1), in particular on the respective lower and upper areas of said storage means (1.1).

The storage means (1.1) also comprise a first outlet (1.1.3) allowing the extraction of an outgoing fluid (FS), in this case, a mixture of water and ozonated water, with said outgoing fluid (FS) being obtained inside the tank comprising the storage means (1.1).

Figure 1 also shows control means (1.1.4) located inside the storage means (1.1), which are connected to the elements of the ozonation system (1) in order to regulate them.

These control means (1.1.4) in turn comprise a processor, a memory and measuring means, in this case an ORP probe, for measuring the oxidation-reduction (redox) potential of the outgoing fluid (FS). These elements are not shown in figure 1.

Finally, the storage means (1.1) also include diffusion means (1.1.5) inside them.

Said diffusion means (1.1.5) are configured by the placement of the aforementioned first (1.1.1) and second (1.1.2) inlets of the storage means (1.1), in addition to also comprising diffuser elements such as a diffuser block.

The storage means (1.1) are in fluidic communication with circulating means (1.2) through the first outlet (1.1.3) of the storage means (1.1).

That is, the first outlet (1.1.3) makes it possible to extract the outgoing fluid (FS) to the circulating means (1.2), in this case a centrifugal pump, so it can be pumped to other elements of the ozonation system (1).

In particular, the circulating means (1.2) direct the outgoing fluid (FS) stream to the mixing means (1.3), in particular a static mixer (1.3).

This outgoing fluid (FS) stream enters the mixing means (1.3) through a first inlet port (1.3.1) and mixes with an ozone stream (FO3), which enters the mixing means (1.3) through a second inlet port (1.3.2).

This mixture makes it possible to obtain an ozonated fluid (FOZ) stream, which is extracted from the mixing means (1.3) through a first outlet port (1.3.3) and directed to the second inlet (1.1.2) of the storage means (1.1) in order to mix with the volume of fluid contained inside said storage means (1.1).

Thus, the ozone (03) concentration of the volume of fluid contained in said storage means (1.1) is enriched by replacing a volume of fluid contained in the tank with a volume of ozonated fluid (FOZ) that has enriched its diluted ozone concentration in the mixing means (1.3).

The mixing means (1.3) in turn comprise a recirculating system (1.3.4) that reintroduces a quantity of ozonated fluid (FOZ) stream into the mixing means (1.3) through the first inlet port (1.3.1) thereof, with said ozonated fluid (FOZ) stream being extracted from the mixing means (1.3) through the first outlet port (1.3.3) thereof in order to further enrich the ozone concentration thereof in the latter.

As shown in figure 1, the ozone stream (FO3) introduced into the mixing means (1.3) through the second inlet port (1.3.2) thereof is obtained by the ozone generating means (1.4), which comprise a reactor (1.4.1) and a concentrator (1.4.2).

The concentrator (1.4.2) comprises a first inlet (1.4.2.1) allowing an ambient air stream (FA) from the outside to enter the concentrator (1.4.2.1) and a second inlet (1.4.2.2) that produces a pure oxygen stream (FO2) after passing through the concentrator (1.4.2).

This pure oxygen stream (FO2) is in turn introduced into the reactor (1.4.1) through the first access thereof (1.4.1.1). After this pure oxygen flow (FO2) passes through the reactor (1.4.1), an ozone stream (FO3) is obtained through the second access (1.4.1.2) thereof.

In this way, the ozone generating means (1.4) make it possible to introduce the ozone stream (FO3) through the second inlet port (1.3.2) of the mixing means (1.3) in order to enrich the outgoing fluid (FS) introduced through the first inlet port (1.3.1).

Additionally, figure 1 also shows a first fluid flow means (1.5) located between the circulating means (1.2) and the first inlet port (1.3.1) of the mixing means (1.3), as well as second fluid flow means (1.6) located between the ozone generating means (1.4) and the second inlet port (1.3.2) of the mixing means (1.3). Said first (1.5) and second (1.6) fluid flow means make it possible to regulate the entry of the respective fluids into the mixing means (1.3).

In this way, the ozonation system (1) makes it possible to extract a volume of fluid from inside the storage means (1.1) and replace it with a volume of ozonated fluid (FOZ) extracted from the mixing means (1.3), so that the contents of said storage means (1.1) are enriched with ozone until a fluid with a suitable ozone content is obtained, with the ozone concentration (CO3) diluted in the fluid, in this case in water, already being predetermined.

Once this optimum ozone concentration (CO3) is reached, a final ozonated fluid (FOZF) volume is obtained inside the storage means (1.1).

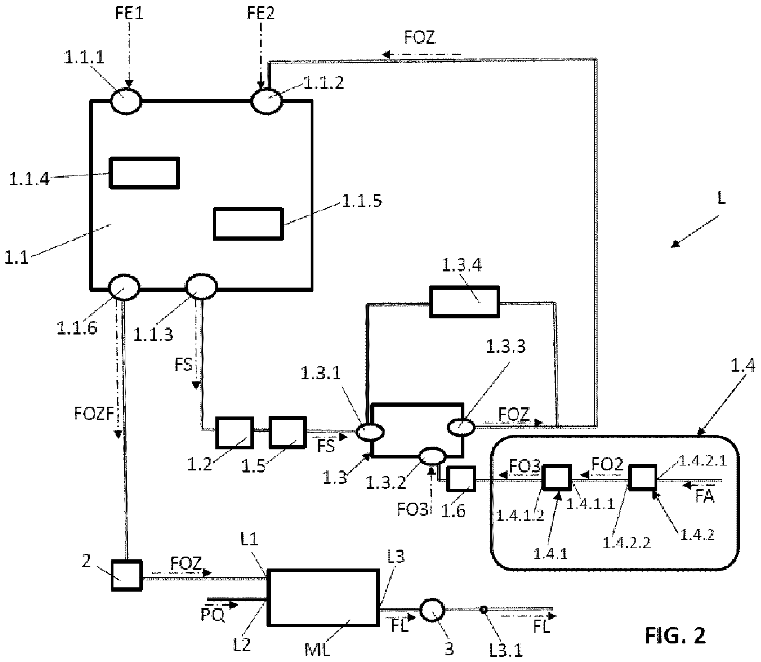

Figure 2 shows a schematic view of a washing system (L) for vehicles according to a first embodiment of the invention, comprising the ozonation system (1) shown in figure 1.

In this case, the figure shows how the storage means (1.1) of said ozonation system (1) also comprise a second outlet (1.1.6) that makes it possible to extract a final ozonated fluid (FOZF) stream through some feeds (2), in particular a centrifugal pump.

This final ozonated fluid (FOZF) stream enters the washing means (ML) through a first washing inlet (L1), drawn from the storage means (1.1) by the said feeds (2).

In addition, a chemical product (PQ) is also introduced into the washing means (ML) through a second washing inlet (L2).

Figure 2 shows how a volume of washing fluid (FL) obtained by mixing the final ozonated fluid (FOZF) and the chemical product (PQ) introduced into the washing means (ML) is extracted from said washing means (ML) through a washing outlet (L3) comprising a nozzle (3.1).

In addition, Figure 2 also shows pressurising means (3) in the washing system (L), which make it possible to extract the washing fluid (FL) from the washing means (ML) at an optimal pressure for washing any element, particularly of a vehicle.

Finally, it needs to be considered that the present invention is not limited by the embodiment disclosed herein. Other embodiments can be made by persons skilled in the art in light of this description. In consequence, the scope of the invention is defined by the following claims.

## Claims

1. Ozonation system (1) configured to ozonate a fluid, **characterised in that** the ozonation system (1) comprises:
- storage means (1.1) configured to store fluids, which comprise:
• a first inlet (1.1.1) configured to allow a first incoming fluid (FE1) to enter the storage means (1.1),
• a second inlet (1.1.2) configured to allow a second incoming fluid (FE2) to enter the storage means (1.1),
• a first outlet (1.1.3) configured to allow an outgoing fluid (FS) to exit the storage means (1.1), and
• control means (1.1.4), which in turn comprise a processor, a memory and measuring means configured to measure ozone concentration (CO3), wherein the control means (1.1.4) are configured to control the ozone concentration (CO3) of the outgoing fluid (FS),
- circulating means (1.2), which are in fluidic communication with the storage means (1.1) and configured to draw an outgoing fluid (FS) stream through the first outlet (1.1.3) of the storage means (1.1),
- mixing means (1.3), which comprise:
• a first inlet port (1.3.1), which is in fluidic communication with the circulating means (1.2) and configured to introduce an outgoing fluid (FS) stream into said mixing means (1.3),
• a second inlet port (1.3.2) configured to introduce an ozone stream (FO3); and
• a first outlet port (1.3.3) configured to allow an outgoing ozonated fluid stream (FOZ) to exit,
wherein the mixing means (1.3) are configured to introduce an ozone stream (FO3) into the outgoing fluid FS) stream, obtaining an ozonated fluid (FOZ), and
wherein the first outlet port (1.3.3) of the mixing means (1.3) is in fluidic communication with the second inlet (1.1.2) of the storage means (1.1),
wherein the control means (1.1.4) are adapted to compare the ozone concentration (CO3) of the outgoing fluid (FS) of the storage means (1.1) with a predetermined ozone concentration value (VCO3), and to perform the following operations:
- if CO3 < VCO3, the control means (1.1.4):
• direct an outgoing fluid (FS) stream through the circulating means (1.2) to the mixing means (1.3), obtaining an ozonated fluid (FOZ) with a higher ozone concentration (CO3), and
• introduce the ozonated fluid (FOZ) stream with higher ozone concentration (CO3) into the storage means (1.1) through the second inlet (1.1.2) of said storage means (1.1),
- if CO3 = VCO3, the outgoing fluid (FS) is a final ozonated fluid (FOZF) and the control means (1.1.4) prevents said final ozonated fluid (FOZF) from exiting the storage means (1.1) by blocking the first output (1.1.3) of the storage means (1.1).

2. Ozonation system (1) according to the preceding claim, **characterised in that** the storage means (1.1) also comprise a second outlet (1.1.6) configured to allow an outgoing fluid (FS) to exit from said storage means (1.1).

3. Ozonation system (1) according to any of the preceding claims, **characterised in that** the storage means (1.1) also comprise diffusion means (1.1.5) configured to obtain a homogeneous outgoing fluid (FS) by mixing the first incoming fluid (FE1) and the second incoming fluid (FE2).

4. Ozonation system (1) according to any of the preceding claims, **characterised in that** it also comprises ozone generating means (1.4) in fluidic communication with the second inlet port (1.3.2) of the mixing means (1.3) and configured to generate the ozone stream (FO3) to be introduced into said mixing means (1.3).

5. Ozonation system (1) according to claim 4, **characterised in that** the ozone generating means (1.4) comprise a reactor (1.4.1), which in turn comprises a first access (1.4.1.1) and a second access (1.4.1.2),
wherein:
- the first access (1.4.1.1) is configured to allow the entry of a pure oxygen stream (FO2),
- the second access (1.4.1.2) is configured to allow the exit of an ozone stream (FO3); and
- the reactor (1.4.1) is configured to obtain an ozone stream (FO3) from the pure oxygen stream (FO2), which is configured to be introduced into the mixing means (1.3).

6. Ozonation system (1) according to claim 5, **characterised in that** the ozone generating means (1.4) also comprise a concentrator (1.4.2), which in turn comprises a concentrator inlet (1.4.2.1) and a concentrator outlet (1.4.2.2), wherein:
- the concentrator inlet (1.4.2.1) is configured to allow an air stream (FA) at ambient conditions to enter,
- the concentrator outlet (1.4.2.2) is in fluidic communication with the first access (1.4.1.1) of the reactor (1.4.1) and configured to allow a stream, preferably a pure oxygen stream (FO2), to exit,
wherein the concentrator (1.4.2) is configured to obtain a pure oxygen stream (FO2) from the air stream (FA) at ambient conditions, which is configured to be introduced into the reactor (1.4.1) of the ozone generating means (1.4).

7. Ozonation system (1) according to any of the preceding claims, **characterised in that** it also comprises first fluid flow means (1.5) located between the circulating means (1.2) and the mixing means (1.3), wherein said first fluid flow means (1.5) are configured to ensure the first incoming fluid (FE1) and the second incoming fluid (FE2) are continuously fed into the storage means (1.1).

8. Ozonation system (1) according to any of the preceding claims, **characterised in that** the mixing means (1.3) also comprise a first injector located on the first inlet port (1.3.1) and configured to inject the outgoing fluid (FS) stream into the mixing means (1.3) through said first inlet port (1.3.1) and/or a second injector configured to inject the ozonated fluid (FOZ) stream into the injection means (1.3) through said first inlet port (1.3.1).

9. Ozonation system (1) according to any of the preceding claims, **characterised in that** the mixing means (1.3) also comprise a recirculating system (1.3.4) configured to recirculate at least a portion of the ozonated fluid (FOZ) stream from the first outlet port (1.3.3) to the first inlet port (1.3.1) of said mixing means (1.3).

10. Ozonation system (1) according to any of the preceding claims, **characterised in that** it also comprises second fluid flow means (1.6) located on the second inlet port (1.3.2) of the mixing means (1.3) and configured to regulate the access of the ozone stream (FO3) through the second inlet port (1.3.2) of the mixing means (1.3).

11. Ozonation system (1) according to claim 10, **characterised in that** the second fluid flow means (1.6) are a solenoid valve, preferably a three-way valve and/or a non-return valve.

12. Ozonation system (1) according to any one of the preceding claims, **characterised in that** the predetermined ozone concentration value (VCO3) of the outgoing fluid (FS) is preferably between 0.5 - 4 ppm of ozone, more preferably between 0.5 - 3 ppm, and most preferably between 1 - 2 ppm.

13. Ozonation system (1) according to any of the preceding claims, **characterised in that** the first incoming fluid (FE1) of the storage means (1.1) is water.

14. Ozonation system (1) according to any of the preceding claims, **characterised in that** at least one of the first inlet (1.1.1), the second inlet (1.1.2) and the first outlet (1.1.3) of the storage means (1.1) comprises an adjustable opening valve, preferably a self-regulating opening valve, configured to connect to the processor of the control means (1.4) of said storage means (1.1).

15. Ozonation system (1) according to any of the preceding claims, **characterised in that** the circulating means (1.2) are a pump, preferably a centrifugal pump.

16. Ozonation system (1) according to any of the preceding claims, **characterised in that** the ozone stream (FO3) introduced through the second inlet port (1.3.2) of the mixing means (1.3) is ozone in its gaseous state.

17. Washing system (L) configured to wash a vehicle, **characterised in that** it comprises:
- washing means (ML)
- a first washing inlet (L1),
- a second washing inlet (L2),
- a washing outlet (L3) comprising a nozzle (L3.1),
- an ozonation system (1) according to any one of claims 2 or 3 - 16 when depending on claim 2, and
- feeds (2) located between the second outlet (1.1.6) of the storage means (1.1) and the first washing inlet (L1), which are in fluidic communication with both,
wherein:
- the feeds (2) are configured to draw a final ozonated fluid (FOZF) stream from the storage means (1.1) of the ozonation system (1) through the second outlet (1.1.6) and introduce it into the washing means (ML) through the first washing inlet (L1),
- the second washing inlet (L2) is configured to allow a chemical product (PQ) to enter,
- the washing means (ML) are configured to mix the final ozonated fluid stream (FOZF) and the chemical product (PQ) to obtain a washing fluid (FL); and
- the nozzle (L3.1) of the washing outlet (L3) is configured to allow a washing fluid (FL) stream to exit.

18. Washing system (L) according to claim 17, **characterised in that** it also comprises pressurising means (3) configured to increase the pressure at the outlet of the washing fluid (FL) stream.

19. A method of washing a vehicle using a washing system (L) according to any one of claims 17 or 18, **characterised in that** it comprises the following steps:
a) providing the washing means (ML) with a final ozonated fluid (FOZF) stream through the first washing inlet (L1),
b) providing the washing means (ML) with a chemical product (PQ) stream,
c) mixing the final ozonated fluid (FOZF) stream and the chemical product (PQ) stream using the washing means (ML),
d) obtaining a washing fluid (FL) stream, and
e) washing the vehicle with the washing fluid (FL) stream extracted through the nozzle (L3.1) of the washing outlet (L3).

20. A method of washing a vehicle using a washing system (L) according to any one of claims 17 or 18, wherein the final ozonated fluid (FOZF) of step a) is obtained through the ozonation system (1), with said ozonation system (1) being configured to operate continuously until the outgoing fluid (FS) meets the condition CO3 = VCO3.

21. A method of washing a vehicle using a washing system (L) according to any one of claims 19 or 20, wherein the chemical (PQ) is soap, preferably liquid soap.
